# EUROPEAN PATENT APPLICATION

(11) **EP 1 249 786 A2**
(43) Date of publication of application: **16.10.2002**
(21) Application number: 02251869.0
(22) Date of filing: 15.03.2002
(51) Int. Cl.: G06F 19/00

(54) **Method and apparatus for delivering and refilling pharmaceuticals**

(30) Priority: 29.03.2001 US 823188
(71) Applicant: Hewlett-Packard Company, Palo Alto, CA 94304 (US)
(72) Inventor: Greeven, John, Corvallis, OR 97330 (US); Greeven, Jeffrey M., Corvallis, OR 97330 (US); Valley, Jeffrey M., Corvallis, OR 97330 (US)
(74) Representative: Jackson, Richard Eric

(57) **Abstract**

An on-line pharmaceutical ordering system (200; 400) enables the transmission of pharmaceutical orders by the Internet (240). Electronic drug delivery appliances (100) that administer or dispense drugs and supplies are equipped with data network interface (123) circuitry by which refills of supplies can be ordered without patient or doctor intervention. In another embodiment, a medical service provider (210) can electronically order supplies for or by the patient (220). On-line validation insures legitimacy of an order.

## Description

### FIELD OF THE INVENTION

This invention relates to medical devices. In particular, this invention relates to medical devices that are used to dispense maintenance drugs.

### BACKGROUND OF THE INVENTION

Many individuals suffer from chronic health problems, the treatment of which requires regular medication deliveries. Treatment regimens for diseases such as diabetes, asthma, epilepsy, cancer and even allergies, require the regular delivery of precise amounts of medication for the patient's survival. Treating chronic medical disorders often requires the administration of medication over a long period of time, and, according to a treatment regimen specified by a medical professional, such as a physician. Other medical professionals, such as psychiatrists and dentists, also prescribe medication that must be regularly taken over a relatively long time period.

One problem that chronic-illness treatment creates is the need to regularly obtain additional medication. The need to replenish needed medicine is at best, an annoyance which can become a problem if, in addition to having to physically travel to more supplies, a doctor's order or script must also be obtained and presented to a pharmaceutical supplier, such as a drug store or pharmacy. A method and apparatus that simplifies pharmaceutical procurement might improve the quality of life and improve the level of care, for those who suffer from chronic illnesses.

### BRIEF DESCRIPTION OF THE DRAWINGS

Figure 1 shows a simplified block diagram of an intelligent drug delivery appliance.
Figure 2 shows a simplified representation of a system for automatically obtaining pharmaceuticals from a supplier of such products.
Figure 3 shows an example of a secure data message used to order and validate pharmaceutical orders.
Figure 4 shows a simplified flow chart of an example procedure of how pharmaceuticals can be automatically ordered via a data network.
Figure 5 shows a simplified flow chart of the process by which a pharmaceutical refill is solicited automatically.

### DETAILED DESCRIPTION OF THE PREFERRED EMBODIMENT

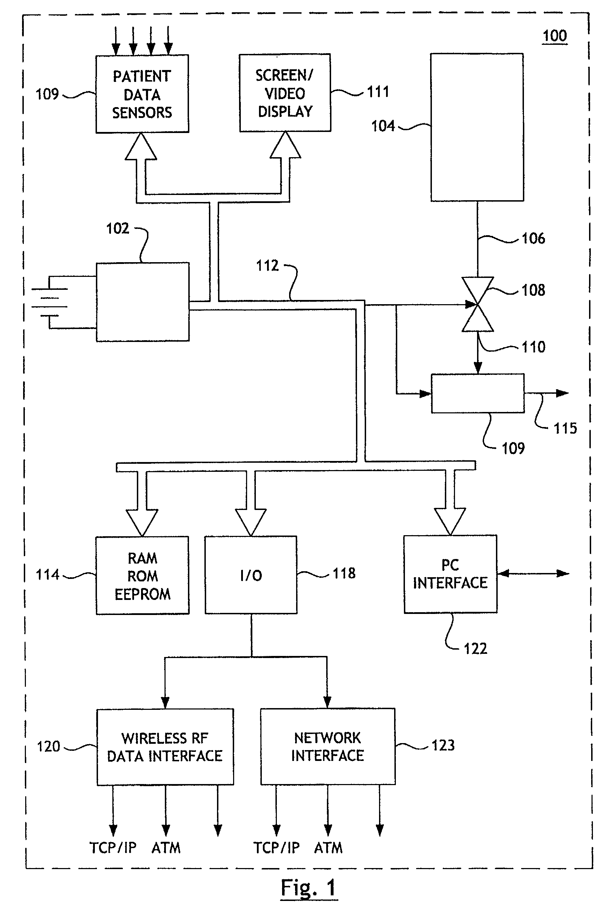

Figure 1 shows a simplified block diagram of an intelligent drug delivery appliance 100 that includes a controlling processor 102 (e.g., a microcontroller, microprocessor, digital signal processor (DSP), combinational/sequential logic and equivalents thereof), operatively coupled to peripheral devices that include, but which are not limited to, a pharmaceutical control valve or dispensing gate 108 of a reservoir of a pharmaceutical 104 through an address/data/control bus 112. By way of example, the reservoir 104 might contain a supply of controlled or medicinal substances such as tablets, liquids, gases, intended to be administered to a patient according to a treatment regimen (i.e. a prescription) of a medical professional (i.e. a doctor, not shown). The reservoir 104 might also store dispensable supplies, such as syringes, reagent test strips (for blood glucose testing for example) antihistamine tablets and the like, also to be used according to some prescribed treatment regiment. For purposes of claim construction, any substance or consumable supply item that might be dispensed to, or used by, a patient is hereafter referred to as a "pharmaceutical." Accordingly, a function of the drug delivery appliance is the dispensing of a pharmaceutical.

One specific example of a pharmaceutical, which might be controllably dispensed, is an aerosol or atomized mist of liquid anti-histamine. By using ink-jet print head technologies, precise amounts of liquids (e.g., antihistamines; insulin) can be controllably dispensed under software control. As the amount of medication is used, the amount remaining in a reservoir can be readily determined. Accordingly, the processor is capable of detecting an impending depletion of a pharmaceutical.

In a drug delivery appliance such as that shown in Figure 1, a treatment regimen (a schedule or circumstance according to which a pharmaceutical is taken by, or administered to a patient from the drug delivery appliance 100) is embodied as computer program instructions (and/or data) stored in a memory device 114 within the appliance. Data parameters that the program operates on, or under the control of, are also stored in a memory device 114. By executing the program instructions, the controller 102 can reliably administer pharmaceuticals according to a doctor's treatment regimen.

By way of example, the program stored in ROM/EEPROM memory 114 (or possibly stored within memory of the processor 102 itself) can effectuate the administration of the aforementioned antihistamine (an example of a "pharmaceutical") from the reservoir 104 to a patient over a predetermined time interval (e.g., hourly, daily, weekly) or, for emergencies, upon patient demand, by opening a valve or gate or other dispensing mechanism 108 for a predetermined amount of time so that a certain amount of the pharmaceutical can be delivered (e.g. flow) from the reservoir 104 to a patient through the valve, (or gate or dispensing mechanism) 108.

In a preferred embodiment, sensors 109 for heart rate, blood pressure, blood sugar, temperature, electrocardiogram, encephalograph signals and waveforms (as well as other patient parameters , are operatively coupled to the processor so as to provide real-time data signals indicative of a patient's physical condition. In such an embodiment, the administration of therapeutic medicines from the reservoir 104 by the processor 102 can then be modulated under software control in response to the information fed back by sensors 109 so as to provide optimal control of a patient's health.

A human/display interface **111** is operatively coupled to the processor 102 via the address/control and data bus 112. Real-time status information (on patient vital signs as well as pharmaceutical availability information or the detection of an operational failure of the drug dispensing appliance) can be displayed to an operator on the human/display interface 111, which could be embodied as a screen such as a CRT or LCD, which for simplicity are generically considered to be the human/display interface 111. Appliance status information (battery status; time of day; diagnostic status) can also be displayed under software control by the processor 102.

As part of the human/display interface, a keyboard or other tactile input device or speech recognition device can be used to input queries to the processor, such as a request to run diagnostic software or to display the amount of pharmaceutical that remains in the reservoir. A keyboard or other input device (e.g., push-button, softkey) can also be used to modify pharmaceutical dosing, providing for example, the capability of delivering an on-demand bolus of pharmaceutical, such as for allergy treatment.

For the visually-impaired, a speech synthesizer can be employed to enunciate statistics and other information that would otherwise be displayed. Speech recognition can be used in place of tactile/switch input devices.

Information, such as the volume of pharmaceutical remaining can be critically important so as to warn can be made available, For purposes of claim construction, all of the foregoing implementations of a human interface are considered to be equivalent "human interface devices."

When the pharmaceutical in the reservoir 104 is depleted, or nears depletion, patient care (and the patient's health) might be jeopardized. In order to help insure that a pharmaceutical supply within the reservoir is never depleted, pharmaceutical refills can be automatically obtained from a pharmaceutical supplier using the methods and apparatus disclosed hereinafter. Once a determination is made that a pharmaceutical needs replenishment, obtaining a refill can be obtained automatically by using data communications exchanged between the drug delivery appliance 100, a medical service provider (e.g., a physician) and a pharmaceutical supplier (i.e. a pharmacy) as described hereinafter. In other words, the intelligent drug delivery appliance can solicit a pharmaceutical refill by way of a data message sent to either a pharmaceutical supplier (e.g., a pharmacy) or to a medical service provider (i.e. a physician) requesting more supplies or an order for more supplies.

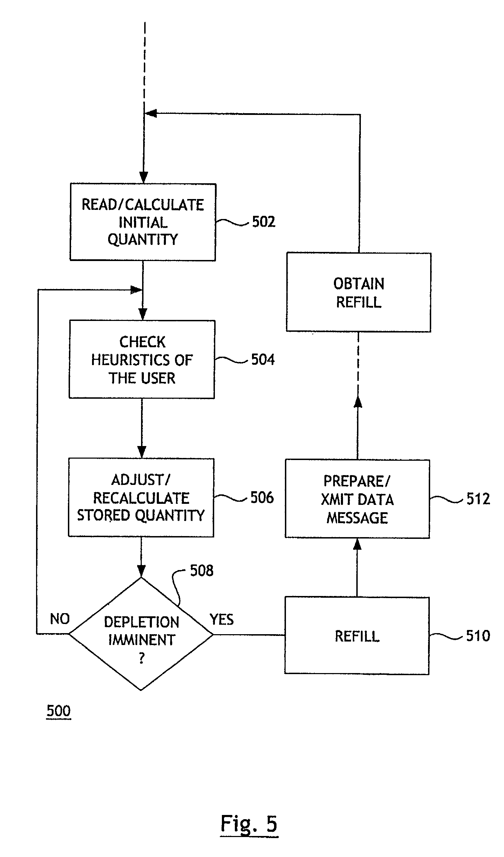

Figure 5 shows a simplified block diagram of steps of a method by which an intelligent drug delivery appliance can dispense pharmaceuticals and automatically obtain refills. In step 502, the processor 102 is apprised of the contents of a reservoir by a data value to the processor 102 or perhaps by a physical measurement. Once the starting contents are known (or believed to be known) in step 504, a pharmaceutical is dispensed, according to a treatment regimen, the parameters of which are provided to the processor 102.

As pharmaceutical is dispensed, the amount of pharmaceutical as determined in step 502 is decremented or otherwise adjusted in step 506. As pharmaceutical is used up, a decision must be made in step 508 whether the amount calculated to be remaining is so low as to warrant the solicitation of a refill in step 5 10 whereupon a data message can be prepared and transmitted in step 5 12 via a data network.

For purposes of this disclosure and claim construction therefore, the process of detecting an impending depletion includes calculation as well as measurement. By virtue of the cooperation between the various components shown in Figure 1 and described herein, for purposes of claim construction, the step of detecting an *impending* depletion should also be considered to include detecting an actual depletion.

The determination of how much pharmaceutical remains in the reservoir 104 might be made in different ways. The amount of pharmaceutical in the reservoir 104 might be identified to the control program running in the processor 102 when the reservoir 104 is filled. As the pharmaceutical is dispensed, calculating the amount remaining in the reservoir by subtraction can yield an amount that remains. By dividing the amount remaining by the amount to be dispensed per unit time, the time at which the reservoir will be depleted can be readily calculated. Other methods by which the pharmaceutical within the reservoir can be determined would include, but not limited to: a measured weight of the reservoir (not shown); a depth measurement by way of an ultrasonic or mechanical transducer (not shown) or measured static pressure within the reservoir 104. The determination of pharmaceutical exhaustion is not germane to an understanding of the invention disclosed and claimed herein. Once a determination is made that more pharmaceutical is required, by using the method and apparatus disclosed hereinafter, obtaining a refill can be automated from either the drug delivery appliance, a medical service provider or a pharmaceutical provider.

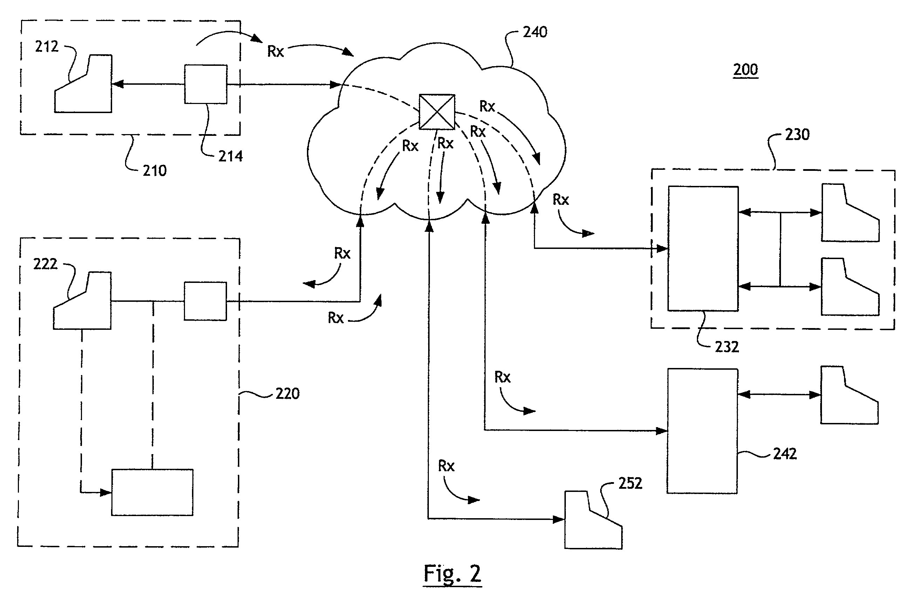

With respect to Figure 2 there is shown a simplified block diagram of a distributed network 200 of computers (such as personal computers, work stations or networks thereof, all appropriately equipped with data network interface capability and Internet browsers) which include: the medical service providers' computer 2 10; patient drug delivery appliances 220 such as those shown in Figure 1 (and identified in Figure 1 by reference numeral 100); and pharmaceutical supplier computers 230, all of which are operatively linked together by a data network 240. As set forth below, computers of insurance providers, governmental agencies 252 or other third parties can also be part of the network 200 shown in Figure 2, by which pharmaceutical supply transactions can be monitored in real time for data related to dispensing, payment, billing and reimbursement transactions. In the preferred embodiment, the data network 240 that links the various computers depicted in Figure 2 includes the now-ubiquitous Internet but might also include local area networks, token rings or other networks by which files can be shared between computers.

It is well known that within the United States and other countries, the sale and/or distribution of some pharmaceuticals is regulated or otherwise controlled by governmental agencies. Obtaining a refill for controlled-distribution pharmaceutical item in such jurisdictions in the U.S. typically requires an order (also known as a "script") from a licensed medical professional, (e.g., a physician) to a pharmaceutical supplier (i.e. a pharmacy or pharmacist) to provide a particular pharmaceutical. Inasmuch as medical professionals in the U.S. are licensed by state agencies, it might be unlawful for a physician in one state to issue a script for a pharmaceutical, in another state. Doctors are therefore generally prevented from prescribing medicines outside the jurisdiction in which they are licensed. In an automated, on-line drug delivery system, insuring compliance with state laws regulating the practice of medicine can become problematic if for instance, the issuance of an electronic prescription by a physician is some how construed to be written by the doctor outside the jurisdiction in which the physician is licensed. Similarly, the transmission of an order or prescription for a pharmaceutical, such as a controlled substance, by an unlicensed or otherwise unauthorized person might subject the pharmaceutical provider to civil and/or criminal liability. By using a secure, electronic data messages to order pharmaceuticals "on-line" unlawful practices can be minimized. For example, by using a unique secure identifier as described hereinafter, a pharmaceutical provider can determine whether an electronic drug order, received via a data network, truly originated from an individual duly licensed to prescribe medication to the patient in the jurisdiction where the pharmaceutical supplier is located or doing business. Presumably, a secure identifier for a medical professional uniquely identifies the professional and is not readily compromised. Upon receipt of a pharmaceutical order accompanied with a unique, secure identifier, a pharmaceutical provider can thereafter provide the item to the patient (by express delivery service, U.S. Postal Service, messenger or the like). In addition to sending pharmaceuticals, a medical service provider's directions (i.e., instructions) on how and when to take or use a particular pharmaceutical can also be delivered thereby providing at least a modicum of assurance that the pharmaceutical will be used appropriately as well as providing some assurance that various state and federal laws controlling the practice of medicine or the distribution sale or of controlled substances, are not violated.

For purposes of claim construction, the term "data message" as well as any other "data" that is sent, received, or exchanged by way of a data network, should not be construed to include facsimile transmissions by which images on a printed page are converted into electronic signals, which are in turn, converted into modulated audio signals for transmission through a telephone network. As used herein, a "data message" is not considered to be a fax or a telephone call but is instead a true data message which a digital computer can send and understand.

With respect to Figure 2, a medical service provider (e.g. a physician, not shown) can initiate the delivery of a pharmaceutical via the Internet (or other data network) by sending a predetermined data message from a personal computer 2 12 (or other data terminal) coupled to the Internet through an appropriate network interface 2 14, such as modem, Ethernet or local area network interface or other equipment by which the computer can send and/or receive data. In a preferred embodiment, an encrypted (and perhaps digitally-signed) data message is sent from the medical service provider's computer 2 12 to one or more pharmaceutical providers' computers 230 via a data message (typically including one or more multi-byte data packets) across the Internet 240. In encrypting and/or digitally signing a data message, the message itself becomes secure and, for purposes of claim construction, is considered to be a secure data message. A "secure prescription data message" is considered to be a secure data message but for the express purpose of obtaining a pharmaceutical using a prescription or script. Upon receipt of the secure data message by the pharmaceutical supplier, the pharmaceutical supplier can elect to fill the order, or in an alternate embodiment, require a separate (second) affirmative approval (i.e, confirmation) message from the putative sender of the pharmaceutical order. For purposes of claim construction, an "approval" message can come from either a health care service provider (e.g. a physician, hospital) as well as a patient. Confirmatory approval messages to confirm a pharmaceutical order can provide assurance that an order for a pharmaceutical was issued by a duly licensed (authorized) practitioner.

A pharmaceutical order approval message can take a variety of forms including a confirmatory data exchange between the pharmaceutical provider and the medical service provider such as the exchange of passwords, state license numbers or the like. Such a confirmatory message might also take the form of a phone call or facsimile message between the medical service provider and the pharmaceutical supplier to establish the true identity of the party ordering a pharmaceutical. Once an order is confirmed using a procedure such as one of the foregoing methods (which, for purposes of claim construction are considered to be processes by which a pharmaceutical order is "validated") the pharmaceutical order can be filled from the pharmaceutical supplier's inventory with an increased assurance that the order is legitimate.

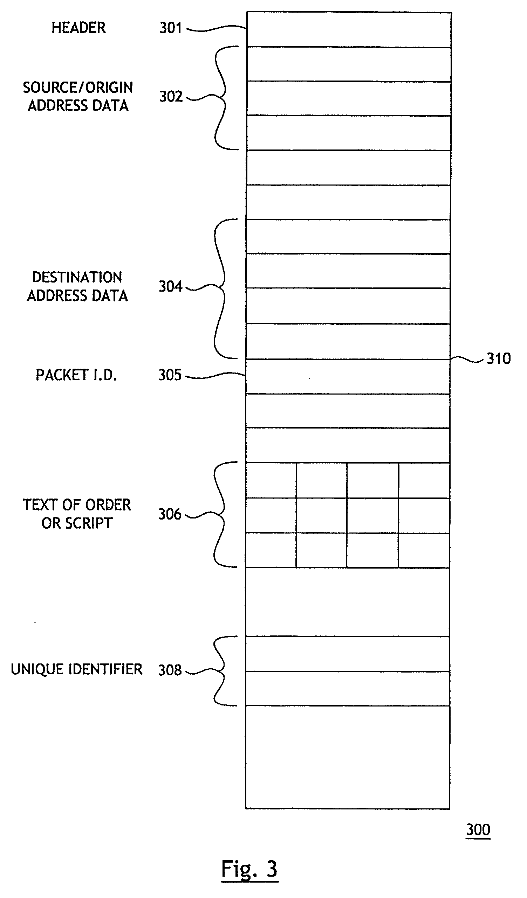

Figure 3 shows an exemplary data message 300 that might be used to order pharmaceuticals over a data network 240, such as the Internet. Those skiiled in the art will recognize that various message formats might be used to securely transmit 'sensitive data over an insecure network. The message 300 of Figure 3 is comprised of several individual data items, in several data fields. A header block 301 is encoded with a particular data value or binary digit (bit) pattern to identify to recipient computers (e.g. 212, 232, 242, 252) that the following data items are a pharmaceutical data message 300. Like an Ethernet data packet, a source address 302 uniquely identifies the computer from which the message originated. A destination address 304 uniquely identifies the computer to which the packet is to be sent. In instances where multiple packets 300 might be sent, a numerical packet identifier 305 might be required so as to be able to reconstruct a multi-packet message if the packets of a multi-packet message are not received in order.

Text of an order or message 306, (e.g. dosage instructions) is followed by a unique data identifier 308 or secure prescription identifier, the purpose of which is to identify the originator of the message. Those skilled in the art of data transmission will recognize that sending a message across a data network such as the Internet can be accomplished in a variety of ways. Moreover, the security of such transmissions can be compromised. The depiction of Figure 3 is only a hypothetical example of how such a message might be formatted. Accordingly, at least one prudent security measure is to encrypt the message 300 and to include some sort of digital signature, either before or after encryption, so as to help insure the safe delivery of the message to its intended recipient.

An encrypted data message from a medical service provider to a pharmaceutical provider (such as that shown in Figure 3) will include at least a lawfully valid prescription or order for a particular pharmaceutical (including dosage instructions). In at least one preferred embodiment, the encrypted data message will include a unique identifier for the medical service provider. By way of example, a unique identifier 308 for a medical service provider might include a multi-digit or multi-character code word (or words) issued by, or generated by and obtained from, a governmental agency, a professional organization or a pharmaceutical provider. In order to maintain it's value as an identifier, a code word identifying a medical service provider is preferably known to only the medical service provider and the issuing entity.

By including a unique identifier with a pharmaceutical dispensing order, a pharmaceutical provider (i.e. a pharmacy or pharmacist) can have at least a modicum of assurance that an incoming electronic order for a controlled pharmaceutical, actually originated with a lawfully-qualified medical service provider, e.g. a medical doctor. Before filling an order, a pharmaceutical provider can have some assurance that the prescribed medication is at the instance of a legitimate health care provider, for a legitimate purpose.

With reference to Figure 2, data transfers across the Internet 240 and between a medical service provider, pharmaceutical providers and patients can take place in a number of ways. One method includes web-hosted communication. By way of example, a pharmaceutical provider might host a web site through which orders for pharmaceuticals can be delivered using hyper text transfer protocol (HTTP) messages. A computer (e.g. a server) that directly or indirectly "hosts" the web site (232 perhaps) can be programmed to accept or read data messages (ASCII strings as well as numeric data) sent to the "web site" by the medical service provider's computer 212 (using a web browser). Another method by which pharmaceuticals might be ordered using data transfers between computer coupled to a data network is by way of "e-mail" messages comprised of data packages, such as the one shown in Figure 3.

In addition to a health care provider sending a data message, automatically obtaining a pharmaceutical refill can also be realized by the patient's intelligent drug delivery appliance sending an electronic pharmaceutical order message 300 to one or more pharmaceutical providers' computers or computer networks 232; 242 via the Internet or other data network 240 requesting a pharmaceutical refill (i.e. analogous to a bidding process). As shown in Figure 2, secure electronic prescriptive orders (Rₓ) can be sent from a medical service provider to the drug delivery appliance (220 in Figure 2), a pharmaceutical supplier 230 or other third-party computer 252, via the data network 240. In much the same way the pharmaceutical scripts are filled today, (i.e. a physician provides a script to the patient, who then obtains the pharmaceutical himself) an electronic pharmaceutical order can originate from a physician or other health care service provider computer 2 12 and then be transmitted to the intelligent drug delivery appliance 220. From the drug delivery appliance 220, the electronic order can be sent to via the data network 240 virtually anywhere, including insurance providers 252 or pharmaceutical suppliers 232,242. In such a scenario, validation of an electronic order for a pharmaceutical might be made by the intelligent drug delivery appliance or by the pharmaceutical provider's computers 232, 242, by querying the medical service provider's computer 212 via the data network 240 to confirm or re-establish the validity of the putative order received via the patient (by requesting and receiving a security code).

Like the scenario described above, the intelligent drug delivery appliance might solicit competitive bids from multiple vendors, or might send an explicit order as circumstances warrant. In sending a request for a bid or quote (in the format shown in Figure 3 for instance) to multiple pharmaceutical suppliers 232,242, a patient and/or his doctor might be able to realize economic savings, or better or faster delivery service, preferential payment terms from one or more particular suppliers, or a combination of other inducements upon which a decision to conduct business with a particular pharmaceutical vendor can be made. Depending upon whether the message sent to a vendor from the medical service provider was an explicit order for a refill, or a request for a quotation or bid, upon receipt of the message 300, the pharmaceutical provider(s) can respond to the message originator by an appropriate responsive data message that the order/request message from the service provider was received, including as appropriate, a request for confirmation of the validity of the electronic pharmaceutical order. Terms or conditions responsive to a request for a quote, including price and/or delivery terms upon which the vendor might provide the pharmaceutical can be provided thereafter.

In addition to sending a response to the medical service provider, a pharmaceutical supplier's computer, (232 242) can send a responsive message to the patient's drug delivery appliance (220) or the medical service provider 212 via the network 240 so as to advise both parties of the order 300 and its fulfillment or denial. As set forth above, a responsive message might also require an affirmation from the putative message originator to validate the legitimacy of the order to the supplier. In addition, advisory messages and affirmation requests can also be sent to the intelligent drug delivery appliance 220.

Instead of sending a request for a bid or quote to multiple pharmaceutical suppliers, a medical service provider might instead send a directive order to a particular supplier for a pharmaceutical refill. Upon receipt of the directive message (and validation of the order by way of the unique identifier 308) the pharmaceutical provider(s) can respond to the message originator by an appropriate acknowledgement message followed by shipment of the supplies directly to the patient. In yet another alternate embodiment, the replenishing shipment can be made to the health care service provider.

Automatically obtaining a pharmaceutical refill can also be realized by the patient's intelligent drug delivery appliance sending an encrypted data message 300 to a pharmaceutical providers' computers or computer networks 232; 242 via the Internet (or other data network) 240 requesting a refill. A pharmaceutical order (Rₓ) can be transmitted to a supplier from the intelligent drug delivery appliance 220 that actually originated from the medical service provider's computer 212 and which was routed to the drug delivery appliance 220 via the data network 240. In such a scenario, validation of the order might be made by the intelligent drug delivery appliance or by the pharmaceutical provider's computers 232,242, which might also query the medical service provider's computer 212 via the data network 240 to establish the validity of the putative order received via the patient. By sending electronic orders to multiple vendors, the intelligent drug delivery appliance can solicit competitive bids from multiple vendors, or might send an explicit order as circumstances warrant.

With respect to Figure 1, data communications between a data network 240 (shown in Figure 2) and the intelligent drug delivery appliance is by way of appropriate network interfaces between the drug delivery appliance 100 and the data network 240. In Figure 1, the processor 102 communicates with an input/output port 118, via the address/data/control bus 112. The input/output port 118, which could be any bidirectional interface, in turn provides a communications interface between the processor (executing the stored program that operates the device 100) and the "outside world" that is accessed via at least one of a wireless data interface 120 (typically a two-way radio data link) or a wireline network interface 123, such as an Ethernet (local area network) LAN card or other mechanism of exchanging messages to and from a data network 240 such as the Internet. At least one alternate embodiment of the intelligent drug delivery appliance provides generic serial and parallel interfaces 122 to a personal computer such that the PC (not shown) acts as a de facto network interface to the drug delivery appliance 100.

In the provision of an on-line drug ordering and delivery system, at least one significant aspect of the invention (at least in jurisdictions where the sale and distribution of certain pharmaceuticals is regulated) is order validation and automated approval of the order on the determination of the order validity. Automated approval and automated validation is enabled through the use of a secure prescription identifier or "PID." As set forth above, a unique identifier 308 of the medical service provider provides a modicum of security to an electronic drug order. Other embodiments might include the exchange of pseudo-randomly generated passwords, not unlike the data exchange that takes place in remote keyless entry systems. In such systems, a processor in a key fob executes a predetermined algorithm that generates a multi-digit keyword upon each activation of a pushbutton switch. A duplicate copy of the algorithm that is executed by a host system compares a received multi-digit keyword to a multi-digit key calculated using the same algorithm. If the received keyword matches a calculated keyword, access to a controlled space is granted. Validation of an electronic prescription can be accomplished in a similar fashion.

An electronic prescription in the form of the message shown in Figure 3 might be formatted with a calculated secure identification data. Upon receipt of the order by a supplier 242, the pharmaceutical supplier can re-calculate the secure identification data and upon determining that the received identifier matched a calculated identifier, conclude that the received message is valid.

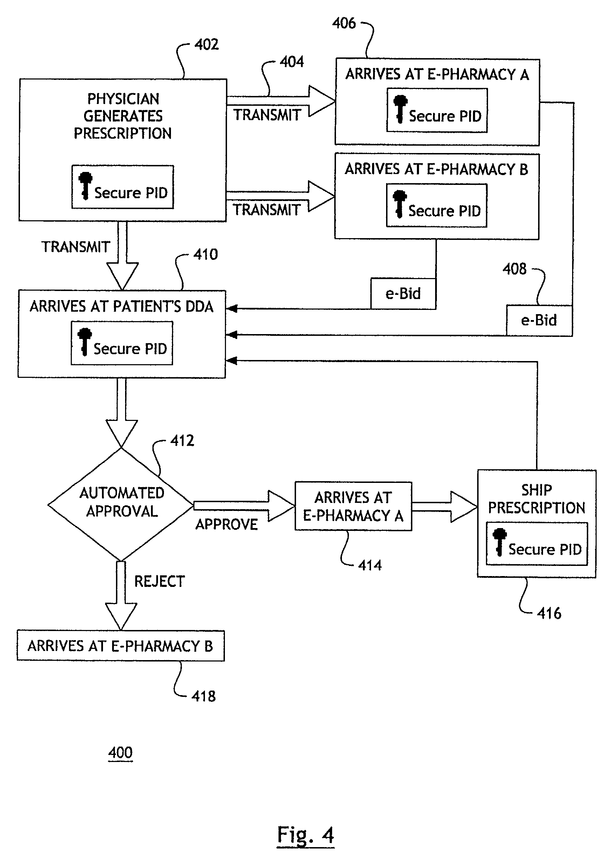

Figure 4 shows a simplified block diagram of the steps of an on-line pharmaceutical order process 400. In step 402, a physician generates an electronic or "e-prescription" message, attaching to it, a validation key or password. In step 404, the e-prescription is sent to one or more pharmaceutical suppliers via a data network using either an e-mail attachment, a web-hosted communications, FTP or other file transfer methodology.

Upon receipt of the e-prescription at step 406, each of the recipient pharmaceutical suppliers can optionally respond with a bid/quote as shown in step 408, or, if the e-prescription was for simply a refill, respond with an affirmation message that the order was received and filled.

In step 4 10, the electronic prescription is shown arriving at a patient's drug delivery appliance. In at least one embodiment, the patient obtains the ability to accept or reject terms offered in an e-bid (step 408) and can thereby choose which supplier to do business with. If a patient prefers, in step 412, software within the drug delivery appliance 100 can automatically provide payment data to the supplier in the form of credit card account numbers or an electronic fund transfer authorization. Prior to paying for offered goods, programming software might set certain minimum conditions as necessary requirements prior to any issuance of payment.

Upon receipt of the payment or authorization or acceptance (in step 414) the pharmaceutical provider can prepare for and execute shipment of the order supplies as depicted in step 4 16.

In step 4 18, a patient might elect to send his own terms and conditions to pharmaceutical suppliers in order to enable the suppliers to decide whether to accept the patient's terms. This sort of reverse auction type of transaction might provide even greater economic benefits to a patient in that pharmaceutical suppliers are required to compete for business upon terms set by the consumer or his health care provider.

Those familiar with medical cost control will appreciate the ability of the method and apparatus disclosed herein to track and control medical costs borne by insurers. As shown in Figure 2, computers of insurance carriers 252 can be included in on-line pharmaceutical order placement and fulfillment by simply addressing messages to them, or copying them with data messages sent to and received by pharmaceutical providers. In such a system, charges for pharmaceuticals that exceed usual, customary or reasonable amounts can be immediately identified. In addition, for those transactions that are paid for by medical insurance, actual payment can be expedited by the electronic transmissions exchanged between doctors, patients and pharmacies. Accordingly, by including an insurance carrier in the electronic transaction, payment might be effected by having the insurer informed of the particulars of the transaction. Furthermore, in so doing, an insurer might modify its benefit provided if for example, generic drugs are supplied instead of so-called name brand pharmaceuticals. All aspects of payment by or reimbursement of expenses by way of transactions provided to an insurer are considered to be in the effort of effecting payment.

By using the nearly instantaneous data transfer capability and nearly ubiquitous availability of the Internet, maintaining a constant supply of health care products can be readily realized. By automatically ordering pharmaceuticals on line using secure, electronic prescriptions, patient care can be kept uninterrupted. In addition, by using secure data transfers as described above, payment for such supplies can also be effectuated by way of electronic data transfers between the parties to such transactions. Insurance providers and governmental regulatory agencies can be included on all aspects of the transaction, expediting reimbursement to the insured and providing for governmental monitoring of transactions for regulatory compliance.

## Claims

1. A method of dispensing a pharmaceutical comprising the steps of:
dispensing (504) said pharmaceutical from an intelligent drug dispensing appliance (100) having: a pharmaceutical reservoir (104) and a data network interface (120, 123);
detecting (508) an impending depletion of the pharmaceutical in the reservoir; and,
soliciting (510, 512) a pharmaceutical refill by way of a data message (300) sent via a data network (240) to which said dispensing appliance is coupled via said data network interface.

2. The method of claim 1 wherein said step of soliciting a pharmaceutical refill by way of a data message, is further comprised of the step of: soliciting a pharmaceutical refill by way of a secure data message (300) sent via a data network to which said dispensing appliance is coupled.

3. The method of claim 1 wherein said step of soliciting a pharmaceutical refill by way of a data message order includes the steps of:
sending a data message (300) over a data network (240), from said intelligent drug dispensing appliance (100) to at least one of:
a health care service provider (210);
a pharmaceutical provider (232);
an insurance provider (252);
said data message (300) from said intelligent drug dispensing appliance (100) identifying at least one of: the patient for whom said pharmaceutical medication is required; pharmaceutical cost; the identity of the particular pharmaceutical; the pharmaceutical provider.

4. The method of claim 3 wherein said step of sending a data message order to an insurance provider includes the additional step of the insurance provider (252) authorizing payment for the pharmaceutical refill.

5. A method of electronically dispensing pharmaceuticals comprising the steps of:
transmitting (404) via a data network (240) from a medical service provider (210), to at least one of:
a patient's intelligent drug dispensing appliance (220) and a pharmaceutical provider (232),
a prescription data message (300) for a pharmaceutical to be dispensed to said patient according to a regimen specified by said medical service provider in said secure prescription data message.

6. A method of electronically dispensing pharmaceuticals comprising the steps of:
transmitting (404) from a medical service provider's computer (210) to at least one pharmaceutical provider's computer (232, 242) via a data network (240), a secure prescription data message (300) for a pharmaceutical to be dispensed to a patient, said secure prescription data message including dosage instructions.

7. The method of claim 6 further including the step of sending a data message via a data network from said pharmaceutical provider's computer to a patient's intelligent drug dispensing appliance so as to direct said appliance to administer said pharmaceutical according to instructions of said medical service provider.

8. An intelligent drug dispensing appliance comprising:
a controller (102);
a reservoir (104) of pharmaceutical to be dispensed over time to a patient;
a drug delivery mechanism (108, 109), coupled to, and responsive to the controller and to the reservoir, to dispense a pharmaceutical to a patient from the reservoir in response to signals from said controller;
a data network interface (120, 122, 123) coupled to said controller.

9. The intelligent drug dispensing appliance of claim 8 including a data network interface (123) that is capable of sending a data message (300) over a data network (240) through said data network interface to at least one of:
a health care service provider (210);
a pharmaceutical supplier (232);
said data message (300) from said intelligent drug dispensing appliance identifying the patient for whom said pharmaceutical is required and the identity of the particular medication.

10. The intelligent drug dispensing appliance of claim 8 including a data network interface (123)that is capable of receiving a data message over a data network through said data network interface from at least one of:
a health care service provider (210);
a pharmaceutical supplier (232, 242);
said data message (300) to said intelligent drug dispensing appliance identifying the patient for whom said pharmaceutical is required and the identity of the particular medication.
